(19) Europäisches Patentamt
European Patent Office
Office européen des brevets

(11) Veröffentlichungsnummer: **0 010 209**
**B1**

(12) # EUROPÄISCHE PATENTSCHRIFT

(45) Veröffentlichungstag der Patentschrift: **20.01.82**

(51) Int. Cl.³: **C 07 C 175/00**, A 61 K 31/165

(21) Anmeldenummer: **79103683.3**

(22) Anmeldetag: **28.09.79**

(54) **N-Benzoyl-retinylamine, Verfahren zu ihrer Herstellung, diese enthaltende pharmazeutische Mittel und die Verwendung dieser Verbindungen bei der Therapie und Prophylaxe von Praekanzerosen und Karzinomen der Haut.**

(30) Priorität: **07.10.78 DE 2843915**

(43) Veröffentlichungstag der Anmeldung: **30.04.80 Patentblatt 80/9**

(45) Bekanntmachung des Hinweises auf die Patenterteilung: **20.01.82 Patentblatt 82/3**

(84) Benannte Vertragsstaaten: **AT BE CH DE FR GB IT LU NL SE**

(56) Entgegenhaltungen:
**AT-B-303 274**
**AT-B-331 426**
**DE-A-2 224 706**

(73) Patentinhaber: **BASF Aktiengesellschaft, Carl-Bosch-Strasse 38, D-6700 Ludwigshafen (DE)**

(72) Erfinder: **Paust, Joachim, Dr., Ringstrasse 3, D-6701 Neuhofen (DE)**
Erfinder: **Nuerrenbach, Axel, Dr., Koenigsberger Strasse 7, D-6718 Gruenstadt 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 209 B1

ACTORUM AG.

# N-Benzoyl-retinylamine, Verfahren zu ihrer Herstellung, diese enthaltende pharmazeutische Mittel und die Verwendung dieser Verbindungen bei der Therapie und Prophylaxe von Praekanzerosen und Karzinomen der Haut

Die vorliegende Erfindung betrifft neue N-Benzoyl-retinylamine der Formel I

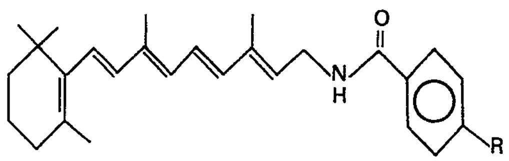

in der R ein Wasserstoffatom oder Methyl bedeutet, ihre Herstellung, diese enthaltende pharmazeutische Zubereitungen und ihre Verwendung als Arzneimittel.

Die Synthese von N-Acetyl- und N-Formyl-retinylamin ist aus der Literatur bekannt (beispielsweise F. Kienzle, Helv. Chim. Acta 56, 1662 ff (1973)).

Weiterhin ist beschrieben, dass sich N-Acetylretinylamin zur systemischen und topischen Therapie von Praekanzerosen und Karzinomen sowie zur systemischen und topischen Prophylaxe von Karzinomen eignet (M.B. Sporn et. al. Nature 263, 110–113 (1976)).

Die celluläre Toxizitätsschwelle für das N-Acetyl-retinylamin liegt dabei oberhalb einer Konzentration von $10^{-5}$ molar («toxicity to tracheal cartilage in vitro»,

Nature 263, 110–113 (1976)), jedoch weist dieses Retinoid im biologischen Versuch am durch Vitamin A-Hypovitaminose keratinisierten Hamstertrachealgewebe nur in $9.10^{-9}$ molarer Konzentration Aktivität auf. Die Methodik hierzu kann G.H. Clamon et al Nature 250, 64–66 (1974) oder M.B. Sporn et al Nature 253, 47–50 (1975) entnommen werden. Die Keratinisierung wird als praekanzerotischer Prozess angesehen.

Es wurde nun gefunden, dass die N-Benzoylretinylamine der Formel I besonders wertvolle pharmakologische Eigenschaften aufweisen, da sie einen wesentlich grösseren therapeutischen Index besitzen.

Bei vergleichbar niederer cellulärer Toxizität verhindern die erfindungsgemässen Verbindungen noch in $10^{-10}$ molarer Lösung die Keratinisierung von Hamstertrachealgewebe und sind damit 1 bis 2 Zehnerpotenzen aktiver als das N-Acetylretinylamin.

Wirkung von Benzoyl-retinylamin bei der Verhütung von Brustkrebs bei Ratten

| | Zahl der Ratten mit Tumoren | Zahl der Tumoren | Zahl der Tumoren grösser als | davon grösser als |
|---|---|---|---|---|
| | | | 0,100 g | 1,0 g |
| Unbehandelt | 20/24 (83%) | 60 | 43 | 21 |
| Mit Benzoyl-retinylamin behandelt | 4/12 (33%) | 10 | 4 | 2 |

Weiblichen Sprague-Dawley Ratten wird eine einmalige Dosis Nitrosomethylharnstoff (50 mg/kg Körpergewicht) intravenöse verabreicht. Ein Teil der Tiere erhält ab dem dritten Tag mit der Nahrung Benzyl-retinylamin (2 mmol/kg Nahrung). Alle Tiere werden 12 Wochen nach der Zufuhr des Carzinogens getötet und untersucht. Die Untersuchungsmethodik kann folgender Literatur entnommen werden: C.J. Grubbs, R.C. Moon, M.B. Sporn, and D.L. Newton, Inhibition of mammary cancer with retinyl methyl ether, Cancer Research 37, 599–602 (1977) und Richard C. Moon, Michael B. Sporn et al. N-(4-Hydroxyphenyl)-retinamide, a new retinoid for prevention of breast cancer in the rat, Cancer Research 39, 1339–1346 (1979).

Die tumorhemmende Wirkung der erfindungsgemässen Verbindungen ist signifikant. Man beobachtet eine Wachstumskontrolle bei in vitro kultivierten Zellen, die beispielsweise nach der Methodik, wie sie von R. Lotan et al im Journal of the National Cancer Institute 60 (1978) Seiten 1035–1041 beschrieben wird, nachgewiesen werden kann. Die erfindungsgemässen Verbindungen inhibiieren die Proliferation von spontan chemisch oder durch Viren transformierter Zellen in Gewebekultur, vorzugsweise verwendet man die S 91 Melanoma Zellinie.

Dementsprechend sind ein weiterer Gegenstand der Erfindung pharmazeutische Mittel, die als Wirkstoff eine Verbindung der Formel I neben pharmazeutisch üblichen Trägerstoffen oder Verdünnungsmitteln und gegebenenfalls üblichen pharmazeutisch-technischen Hilfsstoffen enthalten, und die Verwendung einer Verbindung der Formel I zur Herstellung eines Arzneimittels.

Mittel, welche die erfindungsgemässen Verbindungen der Formel I enthalten, eignen sich bevorzugt zur topischen und systemischen Therapie von Praekanzerosen und Karzinomen der Haut, Schleimhäute und innerer Organe sowie zur systemischen und topischen Therapie von Akne, Psoriasis und anderen mit verstärkter oder pathologisch veränderter Verhornung einhergehenden dermatologischen Erkrankungen. Die erfindungsgemässen Verbindungen verursachen im Gegensatz zur freien Retinsäure keine Hautreizung und keine sog. A-Hypervitaminose.

Ein bevorzugtes Indikationsgebiet ist die prophylaktische und therapeutische Behandlung von Praekanzerosen und Tumoren der Blase, der Brustdüse, der Haut und der Schleimhäute.

Die erfindungsgemässen Verbindungen der Formel I werden in an sich üblicher Weise hergestellt durch Umsetzung von Retinylamin mit einem funktionellen Derivat der Benzoesäure oder der p-Toluylsäure. Als funktionelle Säurederivate eignen sich Ester, Anhydride und bevorzugt Säurehalogenide, insbesondere die Säurechloride. Die Umsetzung wird zweckmässig in Gegenwart

einer Base, insbesondere eines tertiären Amins, vorzugsweise von Pyridin, in einem inerten Lösungsmittel, wie einem Dialkylether oder einem chlorierten Kohlenwasserstoff, vorzugsweise in Diethylether oder Methylenchlorid, durchgeführt. Zweckmässigerweise arbeitet man unter Ausschluss von Sauerstoff und Feuchtigkeit, z.B. unter Stickstoff als Inertgas.

Die Aufarbeitung der Endprodukte erfolgt in an sich üblicher Weise.

Die Ausgangsverbindung all-E-Retinylamin kann nach der o.a. Literaturvorschrift (F. Kienzle) aus all-E-Retinol über das all-E-Retinyl-phthalimid hergestellt werden. Ein weiterer vorteilhafter Syntheseweg für das N-Retinyl-phthalimid besteht in der Wittig-Olefinierung des bekannten $C_{15}$-Phosphoniumsalzes II mit 2-Methyl-4-phthalimido-but-2-en-1-al III.

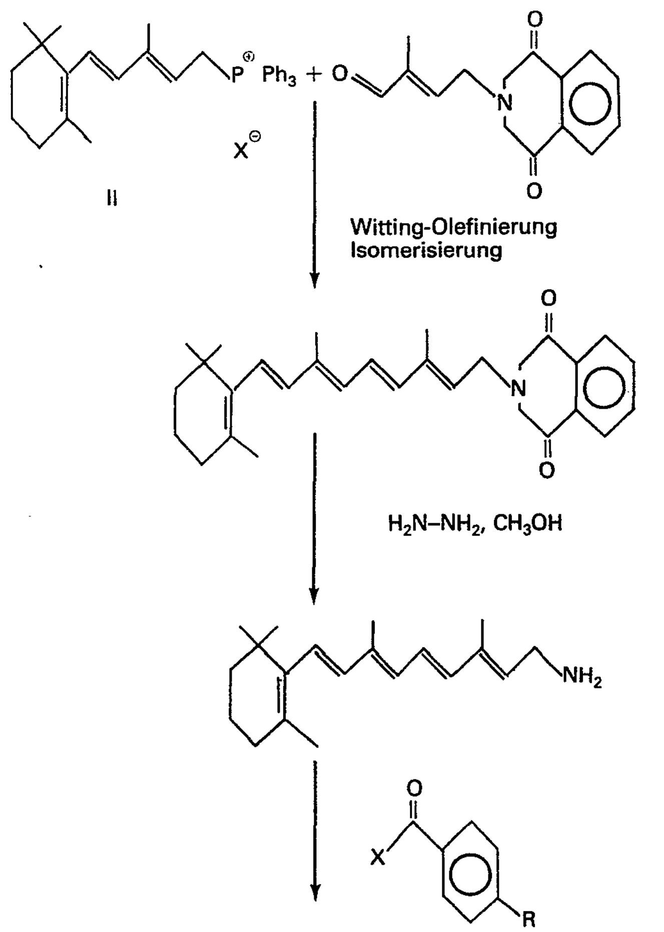


II

III

I

Zur vorliegenden Erfindung gehört auch die Herstellung der therapeutischen Mittel oder Zubereitungen, die mit üblichen Trägerstoffen oder Verdünnungsmitteln und gegebenenfalls üblichen pharmazeutisch-technischen Hilfsstoffen, entsprechend der gewünschten Applikationsart mit einer zur Anwendung geeigneten Dosierung, in an sich üblicher Weise insbesondere durch Vermischen erfolgt.

Die therapeutischen Mittel enthalten die erfindungsgemäss zu verwendenden Verbindungen bei lokaler Anwendung in 0,001 bis 1,0%iger Konzentration, bevorzugt in 0,01 bis 0,1%iger Konzentration, und bei systemischer Anwendung vorzugsweise in einer Einzeldosis von 0,1 bis 5 mg. Dabei kommen als Tagesdosen 5 bis 100 mg in Betracht, wobei die Dosierungen je nach Art und Schwere der Erkrankung, der Zubereitung und der Applikation variieren können.

Es werden die üblichen galenischen Zubereitungen verwendet, für die orale Applikation beispielsweise Tabletten, Filmtabletten, Dragees, Kapseln, Pillen, Pulver, Granulate, Lösungen oder Suspensionen. Für die äusserliche Anwendung kommen insbesondere Pasten, Salben, Gele, Cremes, Lotionen, Puder, Lösungen oder Emulsionen und Sprays in Betracht.

Die erfindungsgemässen Mittel gelangen insbesondere zur innerlichen und äusserlichen Anwendung. Sie werden vorzugsweise oral oder lokal appliziert.

Üblicherweise verwendete pharmazeutisch technische Hilfsstoffe sind beispielsweise für die lokale Anwendung Alkohole, wie Isopropanol, oxäthyliertes Ricinusöl oder oxäthyliertes hydriertes Ricinusöl, Polyacrylsäure, Glycerinmonostearat, Paraffinöl, Vaseline, Wollfett, Polyäthylenglykol 400, Polyäthylenglykol 400-Stearat sowie äthoxylierter Fettalkohol, für die systemische Anwendung Milchzucker, Saccharose, Propylenglykol und Äthanol, Stärke, Talkum, Polyvinylpyrrolidon.

Weitere übliche Zusätze sind beispielsweise Konservierungsmittel, Antioxydantien, geschmackverbessernde Zusätze, Stabilisierungsmittel, Emulgiermittel, Gleitmittel, Netzmittel usw. Voraussetzung ist, dass alle bei der Herstellung pharmazeutischer Zubereitungen verwendeten Stoffe untoxisch und mit den verwendeten Wirkstoffen verträglich sind (vgl. L.G. Goodman, A. Gilman, The Pharmacological Basis of Therapeutics).

Herstellungsbeispiel 1

a) N-(E-3-Formyl-but-2-en-1-yl)-phthalimid:

210 Gewichtsteile 4-Chlor-2-methyl-2-butenal-diethylacetal werden tropfenweise zu einer Lösung von 343,2 Gewichtsteilen ortho-Ameisensäure-ethylester in 151 Gewichtsteilen Ethanol gegeben. Die Lösung wird 1 Stunde bei 40 °C gerührt und bei 16 Torr/40 °C am Rotationsverdampfer eingeengt. Man setzt 400 Gewichtsteile Dimethylformamid und 300 Gewichtsteile Phthalimid-kalium zu, erhitzt 1 Stunde auf 120 °C und filtriert nach dem Abkühlen. Das Filtrat wird am Rotationsverdampfer bei 2 Torr/50 °C eingeengt und der Rückstand in 1500 Raumteilen Diisopropylether und 200 Raumteilen Ethanol aufgenommen. Bei ca. 0 °C kristallisieren über Nacht 172 Gewichtsteile N-(E-4,4-diethoxy-3-methyl-but-2-en-1-yl)-phthalimid aus, die abgesaugt und im Stickstoffstrom getrocknet werden. Das Kristallisat wird in 1500 Raumteilen 80%igem wässrigem Methanol aufgenommen, mit ca. 8 Raumteilen conc. Schwefelsäure versetzt (pH 2) und 30 min bei 50 °C gerührt. Man arbeitet in üblicher Weise durch Trennen zwischen Methylenchlorid und Wasser auf und erhält nach dem Umkristallisieren des Rückstands aus Diisopropylketon 102 Gewichtsteile N-(E-3-Formyl-but-2-en-1-yl)-phthalimid Fp. 112 °C.

b) N-all-E-Retinyl-phthalimid:

Eine 30 gewichtsprozentige Lösung von 1180 Raumteilen β-Jonylidenethyl-triphenyl-phosphonium-hydrogensulfat in Dimethylformamid versetzt man mit 142,6 Gewichtsteilen N-(E-3-Formyl-but-2-en-1-yl)-phthalimid und leitet bei −20 °C bis zur Sättigung Ammoniak ein. Man lässt das Gemisch sich auf ca. 25 °C erwärmen, setzt 1 500 Raumteile n-Hexan zu und wäscht dreimal mit je 650 Raumteilen 60%igen wässrigem Methanol. Die n-Hexanlösung, wird auf ca. 700 Raumteilen eingeengt.Nach dem Abkühlen im Eisbad fallen 115 Gewichtsteile N-all-E-Retinyl-phthalimid in kristalliner Form aus; Fp. 103,5 °C. Weitere 88 Gewichtsteile können nach Isomerisierung aus der Mutterlauge gewonnen werden.

c) N-Benzoyl-all-E-Retinylamin:

16,8 Gewichtsteile N-all-E-Retinyl-phthalimid und 6,2 Raumteile Hydrazinhydrat werden gelöst in 234 Raumteilen Methanol, 2 Stunden unter Rückfluss erhitzt und dann bei 0 °C mit 78 Raumteilen 2 normaler Salzsäure versetzt. Man filtriert das ausgefallene Phthalhydrazid ab, setzt dem Filtrat 1500 Raumteile Wasser zu, extrahiert zweimal mit 250 Raumteilen Ether und setzt dann der wässrigen Phase 80 Raumteile conc. Ammoniak zu. Die wässrig alkalische Phase wird viermal mit je 250 Raumteilen Ether extrahiert. Die übliche Aufarbeitung der Etherphase liefert 11 Gewichtsteile rohes all-E-Retinylamin als rotes Öl.

Das rohe Retinylamin wird in 50 Raumteilen Pyridin bei −20 °C gelöst und tropfenweise mit 5 Gewichtsteilen Benzoylchlorid versetzt. Man lässt auf 20 °C sich erwärmen, setzt 80 Gewichtsteile Eis zu und extrahiert fünfmal mit je 80 Raumteilen Methylenchlorid. Übliche Aufarbeitung liefert 16,8 Gewichtsteile rotes viskoses Öl, das in 50 Raumteilen Diisopropylether aufgenommen wird. Bei −10 °C kristallisieren 5,5 Gewichtsteile N-Benzoyl-all-E-Retinylamin, Fp. 105,5 °C. Die Mutterlauge wird eingeengt (11 Gewichtsteile) und an 300 Gewichtsteilen Kieselgel chromatographiert. Die DC-analytisch reinen Fraktionen liefern nach Kristallisation aus 35 Raumteilen Diisopropylether weitere 3,3 Gewichtsteile an reinem Produkt; Fp. 104 °C.

Herstellungsbeispiel 2

Analog der in Beispiel 1c angegebenen Vorschrift erhält man ausgehend von p-Toluylchlorid das N-p-Toluyl-all-E-retinylamin, Fp. 119,5 °C.

Geeignete pharmazeutische Zubereitungen oder Arzneistoffträger für die äusserliche Anwendung sind z.B.:

Beispiel 1

Lösung

| | | |
|---|---|---|
| N-Benzoyl-all-E-retinylamin | | 0,25 g |
| oxäthyliertes hydriertes Ricinusöl (Cremophor RH 40, Hersteller BASF AG, Ludwigshafen) | | 35,0 g |
| Polyäthylenglykol 400 | | 35,0 g |
| oxäthyliertes Ricinusöl (Softigen 767, Hersteller Chemische Werke, Witten) | | 10,0 g |
| demineralisiertes Wasser | ad | 100,0 g |

Chremophor RH 40 und Softigen 767 werden gemischt und auf 70 °C erhitzt. Die Wirksubstanz wird unter Rühren gelöst und Polyäthylenglykol 400 zugesetzt. Die Lösung wird dann auf 40 °C abgekühlt und unter Rühren wird langsam auf 40 °C erhitztes Wasser zugesetzt. Die fertige Lösung wird filtriert und in z.B. 100 ml Flaschen gefüllt.

Beispiel 2

Creme

| | |
|---|---|
| N-p-Toluyl-all-E-retinylamin | 0,1 g |
| Butylhydroxytoluol | 0,1 g |
| Glycerinmonostearat | 11,0 g |
| Polyäthylenglykol 400-Stearat | 6,0 g |
| äthoxylierter Fettalkohol | 4,0 g |
| Paraffinöl | 10,0 g |
| p-Hydroxybenzoesäureester (Nipasteril, Hersteller Nipalaboratorium Hamburg) | 0,2 g |
| Parfumöl | 0,1 g |
| demineralisiertes Wasser | ad 100,0 g |

Die Fette werden geschmolzen und die feinstgepulverte Wirksubstanz sowie Butylhydroxytoluol unter Rühren bei 65 °C darin verteilt (Lösung I). Das Wasser wird mit dem Nipaester aufgekocht und auf 65 °C abgekühlt (Lösung II). In kleinen Anteilen wird Lösung II unter gutem Rühren in Lösung I einemulgiert. Nach dem Abkühlen auf 45 °C wird das Parfumöl zugesetzt und die Emulsion unter Rühren auf Zimmertemperatur abgekühlt. Die fertige Creme wird in innenschutzlackierte Tuben abgefüllt.

Beispiel 3
Gel

| | |
|---|---|
| N-Benzoyl-all-E-retinylamin | 0,01 g |
| Butylhydroxytoluol | 0,1 g |
| oxäthyliertes Ricinusöl (Cremophor EL, Hersteller BASF AG, Ludwigshafen) | 35,0 g |
| Isopropanol | 20,0 g |
| Polyacrylsäure (Carbopol, Hersteller Goodrich, Hamburg) | 1,5 g |
| Triäthanolamin | 0,002 g |
| p-Hydroxybenzoesäureester (Nipasteril, Hersteller Nipalaboratorium Hamburg) | 0,2 g |
| demineralisiertes Wasser | ad 100,0 g |

Das Cremophor EL wird auf 60 °C erhitzt, der Wirkstoff und das Butylhydroxytoluol unter Rühren gelöst und das Isopropanol, in dem die Nipaester gelöst wurden, zugemischt (Lösung I). Carbopol wird unter kräftigem Rühren in dem Wasser verteilt (Lösung II). Lösung II wird unter gutem Rühren in kleinen Anteilen zu Lösung I gemischt. Der pH-Wert des Gemisches wird mit Triäthanolamin auf 4,5 eingestellt. Das fertige Gel wird in innenschutzlackierte Tuben abgefüllt.

Für die systemische Anwendung besonders geeignete Zubereitungen oder Arzneistoffträger sind z.B.:

Beispiel 4
Tropfen

| | |
|---|---|
| N-Benzoyl-all-E-retinylamin | 0,1 g |
| Propylenglykol | 25,0 g |
| Äthylalkohol | ad 50,0 g |

Äthylalkohol und Propylenglykol werden miteinander gemischt und die Wirksubstanz unter Erwärmen auf 35 °C und unter Rühren gelöst. Nach Filtration wird die Lösung in dunkle Tropfflaschen abgefüllt.

Beispiel 5
Hartgelatinekapseln

| | |
|---|---|
| N-p-Toluyl-all-E-retinylamin | 1 mg |
| Milchzucher | ad 0,25 g |

Die Bestandteile werden gesiebt, gemischt und auf einer geeigneten Kapselfüll- und -verschlussmaschine in Hartgelatinekapseln der Grösse 2 gefüllt.

**Patentanspruch für den Vertragsstaat:** AT

Verfahren zur Herstellung von Benzoyl-retinylaminen der Formel I

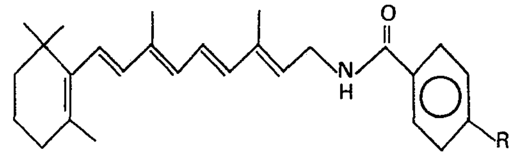

I

in der R Wasserstoff oder Methyl bedeutet, dadurch gekennzeichnet, dass man Retinylamin in an sich üblicher Weise mit einem funktionellen Derivat der Benzoesäure oder p-Toluylsäure umsetzt.

**Patentansprüche für die Vertragsstaaten:**
BE, CH, DE, FR, GB, IT, LU, NL, SE

1. Benzoyl-retinylamine der Formel I

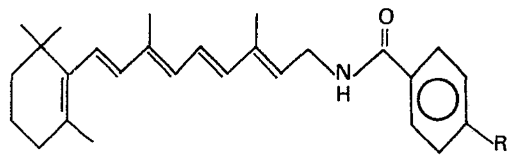

I

in der R Wasserstoff oder Methyl bedeutet.

2. Verfahren zur Herstellung von Verbindungen der Formel I nach Anspruch 1, dadurch gekennzeichnet, dass man Retinylamin in an sich üblicher Weise mit einem funktionellen Derivat der Benzoesäure oder p-Toluylsäure umsetzt.

3. Pharmazeutisches Mittel, gekennzeichnet durch einen Gehalt an einer Verbindung der Formel I nach Anspruch 1 als Wirkstoff neben üblichen Trägerstoffen oder Verdünnungsmitteln und gegebenenfalls pharmazeutisch-technischen Hilfsstoffen.

4. Verbindung der Formel I nach Anspruch 1 zur Verwendung als Arzneimittel bei der topischen und systemischen Therapie und Prophylaxe von Praekanzerosen und Karzinomen der Haut, Schleimhäute und innerer Organe und von dermatologischen Erkrankungen.

**Revendication pour l'Etat contractant:** AT

1. Procédé de préparation de benzoyl-retinylamines de formule I

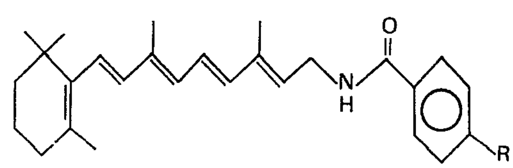

I

dans laquelle R représente hydrogène ou méthyle, caractérisé par le fait qu'on fait réagir de la retinylamine, de manière usuelle en soi, avec un dérivé fonctionnel d'acide benzoîque ou d'acide p-toluylique.

**Revendications pour las Etats contractants:**
BE, CH, DE, FR, GB, IT, LU, NL, SE

1. Benzoyl-retinylamines de formule I

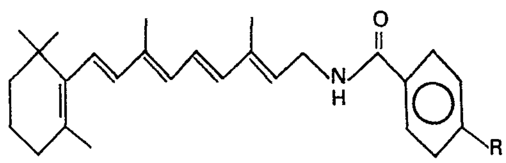

I

dans laquelle R représente hydrogène ou méthyle.

2. Procédé de préparation de composés de formule I selon la revendication 1, caractérisé par le fait qu'on fait réagir de la retinylamine, de manière usuelle en soi, avec un dérivé fonctionnel d'acide benzoïque ou d'acide p-toluylique.

3. Agent pharmaceutique, caractérisé par le fait qu'il contient, comme principe actif, un composé de formule I selon la revendication 1 à côté de supports et de diluants usuels et éventuellement d'auxiliaires pharmaceutiques-techniques.

4. Composé de formule I selon la revendication 1 pour utilisation comme médicament en thérapeutique topique et systémique et prophylaxie des précancéroses et des carcinomes de la peau, des muqueuses et des organes internes et des maladies dermatologiques.

**Claim for the Contracting State:** AT

A process for the preparation of benzoyl-retinylamines of the formula I

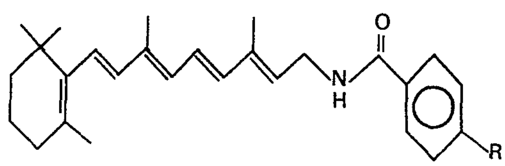

I

where R is hydrogen or methyl, characterized in that retinylamine is reacted in the conventional manner with a functional derivative of benzoic acid of p-toluic acid.

**Claims for the Contracting States:**
BE, CH, DE, FR, GB, IT, LU, NL, SE

1. A benzoyl-retinylamine of the formula I

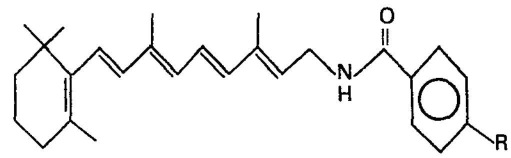

I

where R ist hydrogen or methyl.

2. A process for the preparation of a compound of the formula I as claimed in claim 1, characterized in that retinylamine is reacted in the conventional manner with a functional derivative of benzoic acid or p-toluic acid.

3. A pharmaceutical formulation characterized by a content of a compound of the formula I as claimed in claim 1 as the active ingredient, in addition to conventional carriers or diluents and with or without pharmaceutical auxiliaries.

4. A compound of the formula I as claimed in claim 1 for use as a drug in the topical and systemic therapy and prophylaxis of pre-cancerous conditions and carcinomas of the skin, mucous membranes and internal organs, and of dermatological disorders.